# EUROPEAN PATENT SPECIFICATION

(11) **EP 0 295 041 B1**
(45) Date of publication and mention of the grant of the patent: **22.09.1993**
(21) Application number: 88305163.3
(22) Date of filing: 07.06.1988
(51) Int. Cl.: A61B 17/58

(54) **Apparatus for internal fixation of bone fractures**
Vorrichtung zum internen Fixieren von Knochenfrakturen
Dispositif de fixation interne pour fracture d'un os

(30) Priority: 08.06.1987 CN 87208951; 27.11.1987 CN 87108051
(43) Date of publication of application: 14.12.1988
(73) Proprietor: Zhou, Chonglin, Guoxuexiang Chengdu Sichuan Province (CN); Zhou, Xihua, Guoxuexiang Chengdu Sichuan Province (CN)
(72) Inventor: Zhou, Chonglin, Guoxuexiang Chengdu Sichuan Province (CN); Zhou, Xihua, Guoxuexiang Chengdu Sichuan Province (CN)
(74) Representative: Palmer, Roger

(56) References cited:
- EP-A- 0 024 635
- FR-A- 2 435 243
- US-A- 2 966 907

## Description

The present invention relates to apparatus for internal fixation of bone fractures and, more particularly, to apparatus for internal fixation of bone fractures by using, instead of metal plates, screws and compression tools, a framework structure having teeth thereon for fixing the fractured bone to a satisfied stability and keeping the same automatically compressed by the weight and muscle contraction of the patient per se during the healing of the bone fractures.

In the clinical practice of treating bone fractures, metal plates and screws have been widely used as a typical treatment to internally fix the fractured bone under compression. One such arrangement is shown in FR-A-2435243. Compared with the treatment of external fixation of fractured bone by plaster or splints, better anatomical reduction may be made and a stable and reliable fixation may be achieved at an early stage by compressed internal fixation with metal plates and screws, thereby the patients may be able to get up to do exercises and physical training at an earlier time, therefore avoiding the problems caused by the external fixation, such as muscular atrophy and articular rigidity of disuse.

However, the conventional treatment of the compressed internal fixation with metal plates and screws causes the following unavoidable problems.
1. During the operation, screws must be drilled through the periosteum, bone and marrow, causing serious injuries to the fractured bone and making its structure further damaged and the blood supply to the fractured site further reduced.
2. The mechanical structure of the bone is unbalanced by the screws drilled through causing stress concentration thereabout, and this abnormal stress distribution defers the growing and healing of the fractured bone.
3. From the stand-point of mechanics, when a mechanical system is made up of two or more components that are of different elastic moduli, there occurs in such a system "stress shielding" effect, in other words, when the mechanical system is subjected to a load, the component of higher elastic modulus will carry more of the load than the other to protect or shield the other component of lower elastic modulus, thereby the latter carries less or even no load. Since the elastic moduli of the metal plates and screws are much higher than that of the bone, the plates always perform the stress shielding and protection effects over the bone when they are subjected to stress, thereby the fixation of the fractured bone is protected. However, while the fixation is protected by these stress shielding effects of the plates, the bone-forming activity of the periost is inhibited and the healing of the fractured bone is deferred since the bone and periost at the fractured site are not stimulated by the stress. Furthermore, the lacking of stress will also cause rarefaction of the bone, thus reducing its strength significantly.
4. The conventional internal fixation with plates and screws is conducted at one side of the bone and such a fixation structure is unsymmetrical to the normal state of the bone, hence making the stress subjected by the fractured bone after being fixed also unsymmetrical. The situation is especially serious in the compressed internal fixation, because the pressure over the fractured sits at the side of the plate is greater, while that of the opposite side is smaller and sometimes may even become a harmful separating force (tension). When plates are used on both sides of the fractured bone during fixing, this problem of unsymmetrical structure is not solved thoroughly but causes even more serious compression problems described hereinbelow.
5. When the conventional plates and screws are used for fixation, it is unavoidable that the plates per se form compression along the bone and periost, which compression jeopardize not only the bone-forming activity of the periost but also the blood supply to the fractured site, and cause atrophy in the bone under such compression, the so-called "rarefaction of bone caused by plates."
6. To solve the problem of inadequate stress at the fractured site, the compressed internal fixation is widely used in clinical practice so as to maintain an adequate pressure between tow pieces of bone fractures after they are reduced to promote the healing of the fractured bone. However, since the age, sex, occupation, fractured location of the bone, etc. of patients vary from one to another, it is difficult for the clinical practitioner to decide what will be an adequate value of the pressure for this compressed internal fixation, too much pressure will cause necrosis at the fractured site while too little pressure will defer the healing thereof. In addition, even if adequate pressure is applied to the fractured site during operation, this pressure will be progressively decreased as bone absorption happens at the fractured site during the healing, forming a difficult problem in the internal fixation with plates and screws.
7. Because of drilling, fixing of screws and compressing of two segments of the fractured bone, the operation of internal fixation with plates and screws becomes very complicated since different kinds of tools are needed and doctors should be highly skilled in such operation. All of these mean more damage and more cost to the patient.
8. For above-mentioned reasons, the fractured bone willnot be healed well and the stress distribution ofthe healed bone is not even, sometimes refracture may happen after the plates are removed.

As to the above-mentioned problems, references maybe made to an article entitled "Effects of Stress on Bone and Bone Healing---- some disadvantages of the rigid internal fixation", JOURNAL OF BIOMECHANICS, Vo. 1, No. 1, Jun. 1986, published by Shanghai University of Science and Technology.

In order to solve the above-mentioned problems of the conventional compressed internal fixation with plates and screws, it is disclosed in European patent application No. 80104784.6 (Publication No. 0024635) an internal fixation device for bone fractures comprising a metallic plate having edge fastening formation formed on at least two edges of the plate which is adapted to be secured to a bone fracture site by deforming the plate and engaging the fastening formations directly to the bone by penetration into the bone, thereby bridging a bone fracture. The device has the advantages of avoiding the drilling of screws through the bone, so it has solved the problem of stress concentration caused by the screws, and also avoided the compressing of bone and periost by the plate. However, the device still has the following problems.
1. The stress shielding on the fractured bone caused by the metal plate and the fastening formation thereof is still unsolved.
2. The fixation structure of the device is still unsymmetrical to the normal state of the bone, hence the stress applied to the fractured bone is uneven.
3. The problem of inadequate stress at the fractured site during the internal fixation operation and the healing process of bone fracture is still unsolved, and the pressure between the two segments of the fractured bone will still decrease progressively.
4. The strength and stability of the fixation with this device is not as good as that of the conventional compressed internal fixation with plates and screws.

An alternative device for the fixation of bone structures is disclosed in US-A-2966907. This specification discloses an apparatus for the internal fixation of bone fractures comprising a plurality of elongate stress members, at least two tension members fixed transversely to each end of said stress members to form a framework, each tension member includes at least one detachable coupling structure and one fastening structure, wherein the framework is capable of being detached into two pieces. The apparatus also comprises at least two fastening means for use with said fastening structure of said tension members to close and fasten the same, wherein the internal fixing of the fractured bone is performed.

It has been proved by biomechanic research on the growing of the bone and healing of bone fractures that the various kinds of stress the bone bears decide in some way the external shape as well as the internal structure of the bone. During its life, the bone is growing and changing under the effects of various kinds of stress all the time. Therefore, in the case of bone fractures, the growing and healing of the fractured bone can only be fulfilled with the help of stress.

It is shown by mechanical analysis of the bone that the stress the bone bears under normal state include stress cause by the weight of the body, the contraction of muscle and the load applied to the body by the external environment. These kinds of stress, when applied to the bone, function in the form of pressure, tension, torque, shear and bending, etc, wherein bending may result in pressure on one side of the bone and tension on the other side.

The purpose of the internal fixation of bone fractures is to overcome, with fixing apparatus, all possible kinds of separating force borne by the fractured site, such as tension, torque, and shear, so as to ensure the anatomic reduction and integrity of the fractured bone, promote the healing thereof, and enable the patient to start physical exercises at a very early time to prevent muscular atrophy and articular rigidity. In addition, it has also been proven by biomechanic research that in order to ensure the healing of the fractured bone, it is necessary to apply adequate pressure onto the fractured site to stimulate the growth and healing of the bone. However, because of individual differences, the value of adequate pressure is different for different patients. Yet, adequate pressure for any given person should be the force applied to the bone by his own body weight and/or his own muscle contraction under normal conditions. Therefore, the optimum method for applying a pressure to the fractured site to promote its healing is to use the patient's own body weight and muscle contraction.

One object of the present invention is to provide apparatus for internal fixation of bone fractures comprising a framework structure that offers a balanced fixation of the fractured bone to ensure the anatomic reduction of the fractured bone and the even stress on the fracture site.

Another object of the present invention is to provide apparatus for internal fixation of bone fractures comprising a framework structure which is formed by channel bars for better strength with their bent or concave side facing the bone and with the edges of the channel bars being disposed with teeth structure, therefore, when it is engaged with the bone by the teeth structure, the whole apparatus will not cause plate-compression to the bone and periost.

Yet another object of the present inventin is to provide an internal fixation apparatus for bone fractures which may apply onto the fractured site the pressure caused by the patient's own body weight and muscle contraction by way of specially arranged teeth directions so as to stimulate the growing of the bone and overcome any separating force applied to the fractured bone, thereby promoting its healing and functional recovery.

Apparatus for internal fixation of bone fractures according to the present invention comprises:
a plurality of elongate stress members;
at least two tension members fixed transversely to each end of said stress members to form a framework, each tension member including at least one detachable coupling structure and one fastening structure, wherein the framework is capable of being detached into two pieces;
at least two fastening means for use with said fastening structure of said tension members to close and fasten the same;

wherein, when the internal fixing of the fractured bone is performed, the longitudinal direction of said stress members is kept in the same direction as that of the longitudinal axis of the bone, said stress members being located around the bone at substantially symmetrical positions and said tension members surround the two segments of the fractured bone;
characterised in that said stress members are of a channel led structure opening in use towards the bone and are provided with teeth along their longitudinal edges; said tension members including inwardly facing teeth formed along the two edges thereof; said teeth on said stress and tension members being intended for penetrating into the bone to form a stable engagement therewith.

The apparatus for bone fractures according to the present invention further comprises the following features wherein the teeth of said stress members have one side normal to the edge of said stress member and the direction of the other side of each tooth is inclined to the central part of the stress member which is to lie adjacent the fractured site when fixing is conducted.

When used in treating bone fractures, the apparatus of the present invention has the following advantages:
1. The framework structure of the apparatus guarantees a proper fixation of the fractured bone and the fixing force thereon is evenly distributed with the help of the teeth which penerate into the bone to form a highly stable mechanical system by the apparatus of the present invention together with the fractured bone, which system has good strength against bent, torque and shear, and a light weight, thereby no external fixation with plaster or splints is needed.
2. The damage to the bone and periost caused by the apparatus is very small, no further damage will be made to the marrow or the remaining blood supply at the fractured site, and the operation is simplified, therefore the healing of the fractured bone is promoted.
3. The problems such as stress concentration and stress shielding, caused by the conventional compressed internal fixation with plates and screws are solved and the stress distribution on the fractured bone is improved. Moreover, the patient may start physical exercises and functional training at a very early stage of the treatment so that the fractured site of the bone may be evenly compressed by the patient's own body weight and muscle contraction to stimulate and promote the growing and healing of the fractured bone.
4. During the period of internal fixation with the apparatus of the present invention, a small part of the bone tissues under the compression of the teeth point will be absorbed and after sometime the fixing force applied to the fractured bone by the apparatus through the teeth will gradually decrease, however, at the same time the strength of the bone per se will gradually increase as the bone fracture heals, therefore the stability of the whole system will not decrease and the reduction of the fractured bone will not be changed. On the other hand, the decreasing of the fixing force of the apparatus will enable the bone to bear more stress at the fractured site and this process stimulates and promotes the growth and healing of the fractured bone, thus accelerating the functional recovery of the bone. Such a kind of beneficial process is not possible in the prior art.
5. The apparatus of the present invention may be used in a wide variety of clinical cases, and the internal fixation by its framework structure and teeth engagement may be used to treat different kinds of complicated bone fractures.
6. The framework structure of the apparatus of the present invention forms little shielding to the X-ray, making it easy for the doctors to observe the healing of the fractured bone during the treatment.

The above-mentioned and other objects, features and advantages of the present invention will become more apparent in the following detailed description of the preferred embodiments of the invention made by referring to the accompanying drawings.
Fig. 1 shows the elevation view of the first preferred embodiment of the internal fixation apparatus of the present invention when used in fixing;
Fig. 2 shows the top view of the apparatus shown in Fig. 1;
Figs. 3A-3B show one part of the apparatus shown in Fig. 1;
Figs. 4A-4C show another part of the apparatus shown in Fig. 1;
Figs. 5A-5B show the teeth structure of the stess bar;
Fig. 6 shows illustratively the exploded view of another preferred embodiment of the present invention;
Fig. 7 shows the local top view of the embodiment shown in Fig. 6; and
Fig. 8 shows another embodiment of the stress bar.

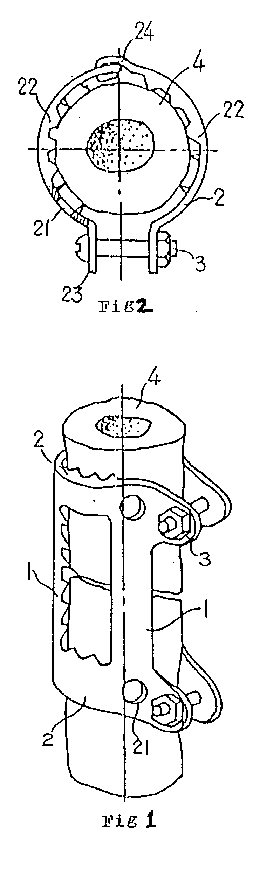

Referring to Fig. 1, there is shown the elevation view of the first preferred embodiment of the apparatus for internal fixation of bone fractures according to the present invention. In Fig. 1, Numeral 1 indicates a longitudinal stress bar. Each apparatus may comprise a plurality of similarly structured stress bars, but preferably three. Numeral 2 indicates a transverse tension ring. Each apparatus may comprise at least two such rings respectively fixed to two ends of the stress bars. The tension ring 2 encloses the fractured bone 4, forming a closed ring, thus a framework structure holding the fractured site of the bone therein is formed by the stress bars 1 and tension rings 2 to provide internal fixation of the fractured bone. Numeral 3 indicates a fastening means by which each tension ring 2 is firmly fastened around the bone with an adequate tension so as to realize stable internal fixation. Obviously, the number of the stress bars in one such apparatus is not limited to three, and that of the tension rings to two, either. The structure of the apparatus may be changed according to actual needs in clinical practice to fulfil the required stability of fixing. In addition, the fastening means shown in Fig. 1 is made up of a screw bolt and a cap, but they may be replaced in practice by any means producing the same effects, such as steel wire, steel belt, rivet, etc. Numeral 21 indicates the openings on the tension ring 2. There are two openings on two ends of the ring so that the operator may fasten the tension ring by holding these two openings with a holding tool during the operation.

Referring to Fig. 2, there is shown the top view of the internal fixation apparatus shown in Fig. 1. Fig. 2 shows the connection of the tension ring 2, its detailed structure and the location of the openings 21 by the local sectional view. Numeral 22 indicates the teeth on the edges of the tension ring 2. When the tension ring 2 is fastened, these teeth 22 are penetrated into the surface of the bone to form a stable engagement with the bone 4. Numeral 23 indicates the fastening structure formed at the ends of the ring 2, which is fastened by the fastening means 3 through this structure. Numeral 24 indicates the coupling structure of the tension ring 2, which is made up of a hook on one side and an opening on the other side. Such a coupling structure is convenient for fixing the whole apparatus onto the fractured bone and removing the same after healing. Obviously, this coupling structure 24 may be replaced by any detachable engagement such as pivotal structure or connecting structure, which will not be further described herein. For the convenience of fixing and removing the internal fixation apparatus, the tension ring 2 shall comprise at least one such detachable coupling structure; of course, more than one such structure will make it more convenient for fixing and removing the whole apparatus.

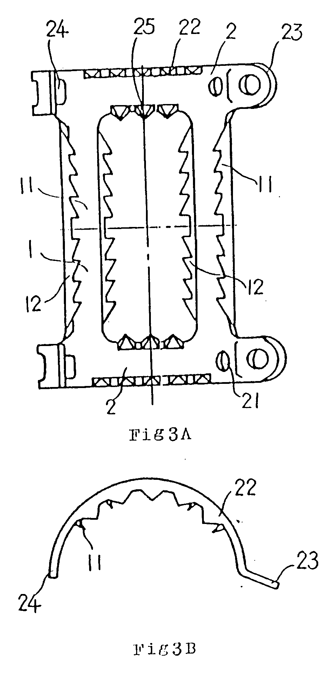

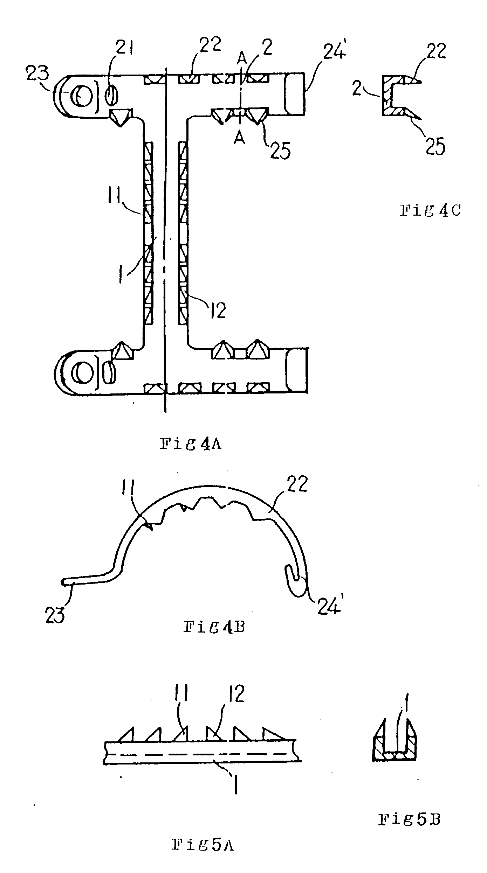

Fig. 3A shows the detailed structure of one part detached from the internal fixation apparatus shown in Figs. 1 and 2, wherein the tension ring 2 has teeth 22 and 25 on its upper and lower edges respectively. The stress bar 1 also has teeth on its two edges, which may be divided along its longitudinal direction into the upper teeth 11 and the lower teeth 12, the direction of the upper teeth 11 being downward and that of the lower ones upward. Their further details are shown in Fig. 5. Fig. 3B, the top view of what is shown in Fig. 3A, shows its curved structure.

Fig. 4A shows the detailed structure of another part detached from the apparatus shown in Figs. 1 and 2, wherein the coupling structure 24' is for connecting with the structure 24 shown in Fig. 3A to form a detachable connection therewith. The structures, functions and numerals of all the other details will not be further described because they are the same as or similar to those shown in Fig. 3A. Fig. 4B shows the top view of that shown in Fig. 4A, and Fig. 4C shows the sectional view made along the dash line A-A shown in Fig. 4A. It is illustrated clearly in Fig. 4C that the teeth 25 are designed to have a downward slope. Such a design, when used in combination with the directional arrangement of the teeth 11 and 12 on the stress bars, will help to apply a pressure to the fractured site.

Fig. 5A shows the detailed structure of the teeth on the stress bar 1. As shown in Fig. 5A, the upper and lower teeth 11 and 12 are all substantially in the shape of right triangle with one right- angled side in normal direction with the edge of the bar 1 and the direction of the slope side being named the direction of the tooth. As shown in Fig. 5A, all the teeth direct to the central part of the stress bar, the place where the fractured side will be when the internal fixing is conducted. It is beneficial to use such centrally-directed teeth structure in fixing because the two segments of the fractured bone are pushed toward each other to be automatically compressed by the patient's own body weight and muscle contraction. Fig. 5B shows the sectional view of the stress bar 1. The channel structure of the bar shown therein increases its bending strength, reduces its own weight and at the same time avoids large-area compression to the bone and the periostium by the bar per se. Obviously, any other concaved, such as U shaped, V shaped, half circle shaped, etc. structures may be used for the same purposes.

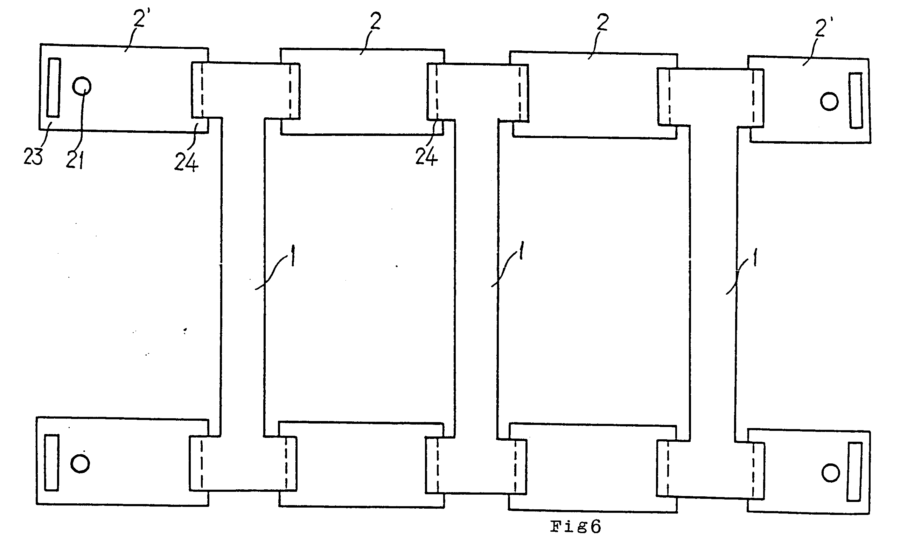

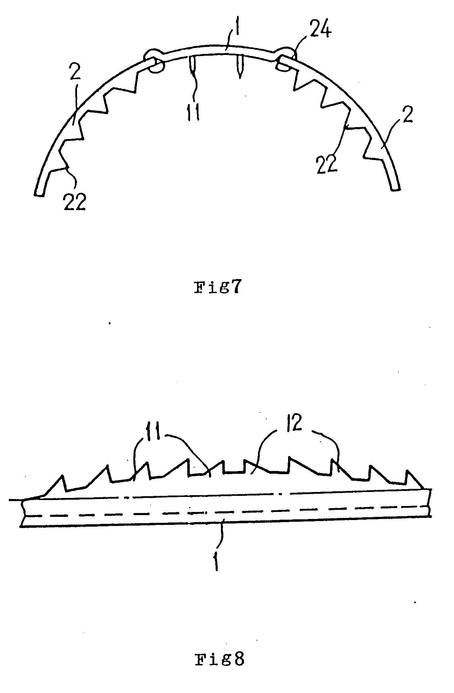

Referring to Fig. 6, there is shown an exploded view of another preferred embodiment of apparatus for internal fixation of bone fractures according to the present invention. In Fig. 6, three stress bars 1 are formed by three I-shaped elements of the same structure, as the central portion thereof being the same with the stress bars shown in Figs 1-5, and with their two ends being provided with the coupling structure of hooks 24 for connecting with the tension element 2. In Fig. 6, each of the tension rings of the apparatus is made up of four tension elements 2 and 2', which are respectivley connected to the ends of the stress bars 1. There are the fastening openings 21 on the tension elements 2' at the two ends. During the operation, the operator may fasten the tension ring around the bone by holding the openings 21 with surgical tools. Each of the element 2' has a fastening structure 23 through which the fastening means such as a steel belt or wire may be used to fix and close the tension ring after it is fastened around the bone. Fig. 7 shows the local top view of the embodiment shown in Fig. 6, wherein two tension elements 2 are respectively connected to the coupling structure at the end of the stress bar 1. The curve and the length of each tension element 2 may be different so that the tension elements of proper sizes may be selected by doctors according to the circumference of the patient's bone. This feature is especially useful for fixing fractured bones haivng different circumferences at the two segments of the fractured bone (the so-called irregular bone's fracture).

Fig. 8 shows another embodiment of the stress bar 1, wherein the teeth are formed along a curved edge so as to fix irregular bone's fractures. Different requirements of fixing bone fractures for different patients and/or at various locations may be met by using elements of different shapes and sizes to form different internal fixation apparatus as that shown in Figs. 6-8. In addition, openings or slots may be made at unstressed part (for example, the central part) of the stress bars and tension elements to further reduce their weight, to help forming micro-circulation between different tissues and to make it convenient for X-ray observation during the healing process.

The internal fixation apparatus for bone fractures according to to the present invention may be made by surgical stainless steel, memory alloy for medical use or any artifical materials for manufacturing artificial bones and articulations. Since all these materials are known in the art and commercially available, they will not be further described herein.

The advantages of the present invention can be easily appreciated by those skilled in the art after reading the detailed description made hereinbefore about different embodiments of the internal fixation apparatus of the present invention in combination with the accompanying drawings.

First of all, the fixing between the present apparatus and the fractured bone is fulfilled via the teeth on the edges of the tension rings and the stress bars, with these teeth penetrating into the surface of the bone to make the fixation stable and the fixing force distributed evenly over the fractured bone. If analysed on any sectional plane of the fixed bone, it will show that the bone is fixed by the teeth of all three stress bars. Such kind of fixation is highly stable and the teeth on the tension rings engaged around the bone at the two ends further increase the capability of the fractured bone against torque. The channel structure of the stress bar has very good bending strength. Therefore, the whole system has very good stability against bending, torque and shear. However, because of the directional arrangement of the teeth on the stress bars and the teeth on the inner edge of the tension rings, when the patient starts physical exercises for functional recovery, the patient's own body weight and muscle contraction will easily affect on the fractured site to provide an adequate sectional pressure for promoting the growing and healing of the fractured bone. The value of such pressure caused by the patient's exercises is just in the optimum range for promoting the bone's healing. Accordingly, the internal fixation apparatus of the present invention accelerates the healing of the fractured bone and enables the same to recover its normal functions and strength in a shorter time.

Obviously, the description with the preferred embodiments of the present invention in combination of the drawings made hereinbefore are only for the purposes of understanding the present invention.

For example, the number of the stress bars is not limited to three, i.e. for a relatively small bone, the number may be two, and steel wires may be used to replace the tension rings for fixing, while for a relatively big bone, the number of the stress bars and tension rings may be increased correspondingly.

## Claims

1. Apparatus for internal fixation of bone fractures, comprising:
a plurality of elongate stress members (1);
at least two tension members (2) fixed transversely to each end of said stress members (1) to form a framework, each tension member (2) including at least one detachable coupling structure (24) and one fastening structure (23), wherein the framework is capable of being detached into two pieces;
at least two fastening means (3) for use with said fastening structure (23) of said tension members (2) to close and fasten the same;
wherein, when the internal fixing of the fractured bone (4) is performed, the longitudinal direction of said stress members (1) is kept in the same direction as that of the longitudinal axis of the bone (4), said stress members (1) being located around the bone (4) at substantially symmetrical positions and said tension members (2) surround the two segments of the fractured bone (4);
characterised in that said stress members (1) are of a channelled structure opening in use towards the bone (4) and are provided with teeth (11,12) along their longitudinal edges; said tension members (2) including inwardly facing teeth (22,25) formed along the two edges thereof; said teeth (11,12,22,25) on said stress (1) and tension (2) members being intended for penetrating into the bone (4) to form a stable engagement therewith.

2. Apparatus as claimed in claim 1, wherein said teeth (11,12) of said stress members (1) have one side normal to the edge of said stress members (1) and the direction of the other side of each tooth (11,12), is inclined to the central part of the stress member (1) which is to lie adjacent the fractured site when fixing is conducted.

3. Apparatus according to claim 1 or claim 2, wherein said teeth (22,25) formed along the inner edge of the tension members (2) slope toward the fractured site.

4. Apparatus according to claims 1, 2 or 3, wherein said fastening means (3) is selected from a group of means including, screw bolt and cap, steel belt, steel wire, and rivet.

5. Apparatus according to any of the preceding claims, wherein said fastening structure (23) of said tension members (2) is formed by a pair of openings at the two ends of said tension members (2) for connecting with said fastening means (3), and a pair of tension openings (21) to be held by the operator with surgical tools in order to fasten said tension members (2) around the bone (4) and mount said fastening means (3).

6. Apparatus according to any of the preceding claims, wherein all parts of the apparatus are made of surgical stainless steel.

7. Apparatus according to any of claims 1 to 5, wherein all parts of the apparatus are made of memory alloy for medical use.

8. Apparatus according to any of claims 1 to 5, wherein all parts of the apparatus are made of materials suitable for making artificial bones and articulations.

9. Apparatus according to any one of claims 1 to 8, wherein:
said stress members (1) have two coupling structures (24) at each end part thereof;
said tension members (2) include a plurality of tension elements (2,2'), each having two coupling structures (24) for connecting with said coupling structures on said end part of said stress means (1); and
wherein, when used in fixing, each tension member (2) is made up of the plurality of said tension elements (2,2') connected between said end part of said stress members (1) to form a closed ring to enclose the fractured bone (4).

10. Apparatus as claimed in any one of claims 1 to 8, wherein said stress members (1) have teeth (11,12) arranged longitudinally along two curved edges so as to fix fractures of irregular bones.

## Patentansprüche

1. Vorrichtung zur inneren Fixierung von Knochenbrüchen mit
einer Mehrzahl länglicher Belastungsglieder (1
wenigstens zwei Spanngliedern (2), die quer an jedem Ende der Belastungsglieder (1) befestigt sind, um so ein Rahmenwerk zu bilden, wobei jedes Spannglied (2) wenigstens eine lösbare Kupplungsstruktur (24) und eine Befestigungsstruktur (23) einschließt, und das Rahmenwerk in zwei Stücke zerlegbar ist;
wenigstens zwei Befestigungsmitteln (3), die zusammen mit der Befestigungsstruktur (23) der Spannglieder (2) verwendbar sind, um diese zu verschließen und zu befestigen;
wobei bei Ausführung der inneren Fixierung des gebrochenen Knochens (4) die Längsrichtung der Belastungsglieder (1) in der gleichen Richtung wie diejenige der Längsachse des Knochens (4) gehalten ist, die Belastungsglieder (1) an im wesentlichen symmetrischen Positionen um den Knochen (4) herum angeordnet sind und die Spannglieder (2) die beiden Segmente des gebrochenen Knochens (4) umschließen;
dadurch gekennzeichnet, daß die Belastungsglieder (1) von einer rinnenförmigen Struktur sind, die sich bei Anwendung zum Knochen (4) hin öffnet, und entlang ihrer Längskanten mit Zähnen (11, 12) versehen sind; die Spannglieder (2) nach einwärts gerichtete Zähne (22, 25) einschließen, die entlang ihrer beiden Kanten ausgebildet sind; und die Zähne (11, 12; 22, 25) an den Belastungs- und Spanngliedern (1 bzw. 2) zu einem Eindringen in den Knochen (4) bestimmt sind, um mit ihm einen stabilen Eingriff auszubilden.

2. Vorrichtung nach Anspruch 1, bei welcher die Zähne (11, 12) der Belastungsglieder (1) eine zu der Kante des Belastungsgliedes (1) senkrechte Seite haben und die Richtung der anderen Seite jedes Zahnes (11, 12) zum Mittelteil des Belastungsgliedes (1) hin geneigt ist, der bei Ausführung der Fixierung in die Nähe der gebrochenen Stelle zu liegen kommt.

3. Vorrichtung nach Anspruch 1 oder 2, bei welcher die Zähne (22, 25), die entlang der inneren Kante der Spannglieder (2) ausgebildet sind, zur gebrochenen Stelle hin schräg gerichtet verlaufen.

4. Vorrichtung nach Anspruch 1, 2 oder 3, bei welcher die Befestigungsmittel aus einer Gruppe von Mitteln ausgewählt sind, die Schraubbolzen und Muttern, Stahlriemen, Stahldraht und Nieten einschließt.

5. Vorrichtung nach einem der voranstehenden Ansprüche, bei welcher die Befestigungsstruktur (23) der Spannglieder (2) durch ein Paar von Öffnungen an den beiden Enden der Spannglieder (2) zur Verbindung mit den Befestigungsmitteln (3) gebildet ist und aus einem Paar von Spannöffnungen (21), die von dem Operateur mit chirurgischen Werkzeugen gehalten werden können, um die Spannglieder (2) um den Knochen herum zu befestigen und die Befestigungsmittel (3) zu montieren.

6. Vorrichtung nach einem der voranstehenden Ansprüche, bei welcher alle Teile der Vorrichtung aus chirurgischem, rostfreiem Stahl gefertigt sind.

7. Vorrichtung nach einem der Ansprüche 1 bis 5, bei welcher alle Teile der Vorrichtung aus Memory-Legierung für medizinische Anwendung gefertigt sind.

8. Vorrichtung nach einem der Ansprüche 1 bis 5, bei welcher alle Teile der Vorrichtung aus einem Material gefertigt sind, das für die Herstellung künstlicher Knochen und Gelenke geeignet ist.

9. Vorrichtung nach einem der Ansprüche 1 bis 8, bei welcher:
die Belastungsglieder (1) zwei Kupplungsstrukturen (24) an jedem ihrer Endabschnitte haben;
die Spannglieder (2) eine Mehrzahl von Spannelementen (2, 2') einschließen, von denen jedes zwei Kupplungsstrukturen (24) zur Verbindung mit den Kupplungsstrukturen an den Endabschnitten der Belastungsglieder (1) einschließt; und
bei welcher bei Anwendung zur Fixierung jedes Spannglied (2) aus der Mehrzahl der Spannelemente (2, 2') aufgebaut ist, die zwischen den Endabschnitten der Belastungsglieder (1) verbunden sind, so daß sie zur Umschließung des gebrochenen Knochens (4) einen geschlossenen Ring bilden.

10. Vorrichtung nach einem der Ansprüche 1 bis 8, bei welcher die Belastungsglieder (1) Zähne (11, 12) aufweisen, die in Längsrichtung entlang zweier gekrümmter Kanten angeordnet sind, um so Brüche unregelmäßiger Knochen zu fixieren.

## Revendications

1. Appareil pour fixation interne de fractures d'os, comprenant une pluralité d'éléments de contrainte (1) allongés ; au moins deux éléments de tension (2) fixés transversalement à chaque extrémité desdits éléments de contrainte (1) afin de former une carcasse, chaque élément de tension (2) incluant au moins une structure d'accouplement (24) séparable et une structure d'assujettissement (23), où la carcasse est capable d'être séparée en deux pièces ; au moins deux moyens d'assujettissement (3) pour utilisation avec ladite structure d'assujettissement (23) desdits éléments de tension (2) afin de fermer et assujettir celle-ci ; où lorsque la fixation interne de l'os (4) fracturé est exécutée, la direction longitudinale desdits éléments de contrainte (1) est maintenue dans la même direction que celle de l'axe longitudinal de l'os (4), lesdits éléments de contrainte (1) étant situés autour de l'os (4) à des positions sensiblement symétriques et lesdits éléments de tension (2) entourent les deux segments de l'os (4) fracturé ; caractérisé en ce que lesdits éléments de contrainte (1) ont une structure rainurée ouvrant en utilisation vers l'os (4) et sont pourvus de dents (11, 12) le long de leurs bords longitudinaux ; lesdits éléments de tension (2) incluant des dents (22, 25) faisant face vers l'intérieur, formées le long des deux bords de ceux-ci ; lesdites dents (11, 12, 22, 25) sur lesdits éléments de contrainte (1) et de tension (2) étant conçus pour pénétration dans l'os (4) afin de former un engagement stable avec celui-ci.

2. Appareil comme revendiqué dans la revendication 1, où lesdites dents (11, 12) desdits éléments de contrainte (1) ont un côté normal au bord desdits éléments de contrainte (1) et la direction de l'autre côté de chaque dent (11, 12), est inclinée vers la partie centrale de l'élément de contrainte (1) qui est à disposer adjacent à l'emplacement fracturé lorsque la fixation est effectuée.

3. Appareil selon la revendication 1 ou la revendication 2, où lesdites dents (22, 25) formées le long du bord intérieur de l'élément de tension (2) s'inclinent vers l'emplacement fracturé.

4. Appareil selon les revendications 1, 2 ou 3 où lesdits moyens d'assujettissement (3) sont sélectionnés à partir d'un groupe de moyens incluant, boulons filetés et chapeau, bande d'acier, fil d'acier et rivet.

5. Appareil selon l'une quelconque des revendications précédentes, où ladite structure d'assujettissement (23) desdits éléments de tension (22) est formée par une paire d'ouvertures aux deux extrémités desdits éléments de tension (2) pour jonction avec lesdits moyens d'assujettissement (3), et une paire d'ouvertures de tension (21) à maintenir par l'opérateur avec des outils chirurgicaux de façon à assujettir lesdits éléments de tension (2) autour de l'os (4) et monter lesdits moyens d'assujettissement (3).

6. Appareil selon l'une quelconque des revendications précédentes, où toutes les parties de l'appareil sont faites d'acier inoxydable chirur- gical.

7. Appareil selon l'une quelconque des revendications 1 à 5, où toutes les parties de l'appareil sont faites d'alliage à mémoire pour usage médical.

8. Appareil selon l'une quelconque des revendications 1 à 5, où toutes les parties de l'appareil sont faites de matériaux convenables pour fabriquer des os et des articulations artificiels.

9. Appareil selon l'une quelconque des revendications 1 à 8, où lesdits éléments de contrainte (1) ont deux structures d'accouplement (24) à chaque partie d'extrémité de ceux-ci ; lesdits éléments de tension (2) incluent une pluralité d'éléments de tension (2, 2'), chacun ayant deux structures d'accouplement (24) pour jonction avec lesdites structures d'accouplement sur ladite partie d'extrémité desdits moyens de contrainte (1) ; et où lorsqu'il est utilisé en fixation, chaque élément de tension (2) est composé de la pluralité desdits éléments de tension (2, 2') joints entre ladite partie d'extrémité desdits éléments de contrainte (1) afin de former une bague fermée afin d'enfermer l'os (4) fracturé.

10. Appareil comme revendiqué dans l'une quelconque des revendications 1 à 8, où lesdits éléments de contrainte (1) ont des dents (11, 12) disposées longitudinalement le long de deux bords cintrés de façon à fixer des fractures d'os irréguliers.
